# EUROPÄISCHE PATENTANMELDUNG

(11) **EP 0 792 861 A1**
(43) Veröffentlichungstag der Anmeldung: **03.09.1997**
(21) Anmeldenummer: 97102843.6
(22) Anmeldetag: 21.02.1997
(51) Int. Cl.: C07C 45/65, C07C 49/83

(54) **Verfahren zur Herstellung von hydroxylierten Benzophenonen**

(30) Priorität: 01.03.1996 DE 19607809
(71) Anmelder: BASF AKTIENGESELLSCHAFT, 67056 Ludwigshafen (DE)
(72) Erfinder: Aumüller, Alexander, Dr., 67435 Neustadt (DE); Brill, Gunter, Dr., 67454 Hassloch (DE); Böttcher, Peter, Dr., 67316 Carlsberg (DE)

(57) **Zusammenfassung**

Herstellung von hydroxylierten Benzophenonen aus ganz oder teilweise methylierten Hydroxybenzophenonen durch Umsetzung dieser Methylether mit AlCl₃ und anschließende Hydrolyse mit Wasser, dadurch gekennzeichnet, daß man die Umsetzung in Gegenwart einer oder mehrerer Verbindungen aus der Klasse der Harnstoffe oder Carbonsäureamide durchführt.

## Beschreibung

Gegenstand der vorliegenden Erfindung ist ein Verfahren zur Herstellung von hydroxylierten Benzophenonen aus ganz oder teilweise methylierten Hydroxybenzophenonen durch Umsetzung dieser Methylether mit AlCl₃ und anschließende Hydrolyse mit Wasser.

Die Abspaltung der Methylgruppen von methylierten Hydroxybenzophenonen ist eine lange bekannte Reaktion. Diese Etherspaltung kann durch verschiedene Sauren, z.B. durch die Lewissäure AlCl₃ katalysiert werden. So wird in US 2 694 729 die Umsetzung von 2,2',4,4'-Tetramethoxybenzophenon in Gegenwart von AlCl₃ und chlorierten Kohlenwasserstoffen zu 2,2',4,4'-Tetrahydroxybenzophenon beschrieben. In der JP-A 58 216 139 wird die gleiche Umsetzung in Gegenwart von AlCl₃ und o-Dichlorbenzol beschrieben. In DE-C1-41 34 773 wird wiederum diese Umsetzung in Gegenwart von AlCl₃ und aromatischen Kohlenwasserstoffen, insbesondere Xylol, beschrieben.

Die Ausbeuten der bekannten Demethylierungsverfahren lassen jedoch zu wünschen übrig. Ursache der unbefriedigenden Ausbeuten ist meist ein Verklumpen des AlCl₃ mit den Edukten und eine Beeinträchtigung der Rührbarkeit des Reaktionsansatzes infolge dieser Verklumpungen.

Die Aufgabe der vorliegenden Erfindung war es daher, ein Verfahren zur Demethylierung methylierter Hydroxybenzophenone zu finden, welches diese Verklumpungen vermeidet und die Hydroxybenzophenone in guten Ausbeuten liefert.

Demgemäß wurde ein Verfahren zur Herstellung von hydroxylierten Benzophenonen aus ganz oder teilweise methylierten Hydroxybenzophenonen durch Umsetzung dieser Methylether mit AlCl₃ und anschließende Hydrolyse mit Wasser gefunden, das dadurch gekennzeichnet ist, daß man die Umsetzung mit AlCl₃ in Gegenwart einer oder mehrerer Verbindungen aus der Klasse der Harnstoffe oder Carbonsäureamide durchführt.

Die Verbindungen aus der Klasse der Harnstoffe oder Carbonsäureamide sind breit variierbar. In der Regel wird man möglichst gut zugängliche und billige Harnstoffderivate oder Carbonsäureamide verwenden. Beispielsweise kommen daher Verbindungen der allgemeinen Formel I 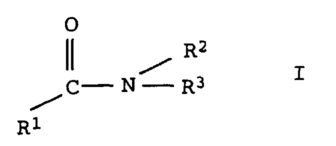 in Betracht, wobei die Variablen die folgende Bedeutung haben:
- R¹: Wasserstoff, C₁-C₄-Alkyl, Amino, Di-C₁-C₄-alkylamino, C₁-C₄-Monoalkylamino, Phenylamino, Diphenylamino, Piperidinyl oder Pyrrolidinyl und
- R², R³: Wasserstoff, C₁-C₄-Alkyl, Phenyl oder C₅- oder C₆-Cycloalkyl oder, gemeinsam mit dem Stickstoffatom, an das sie gebunden sind, einen Piperidinyl- oder Pyrrolidinylrest.

Verbindungen der allgemeinen Formel I sind beispielsweise Harnstoffverbindungen wie N,N'-Dimethylharnstoff, N-Methylharnstoff, Tetramethylharnstoff, N,N'-Dimethylethylenharnstoff, N,N'-Dimethylpropylenharnstoff, Phenylharnstoff, N,N'-Diphenylharnstoff und aus Kostengründen besonders bevorzugt unsubstituierter Harnstoff.

Als Carbonsäureamide der allgemeinen Formel I kommen beispielsweise Formamid, Dimethylformamid, Acetamid, Dimethylacetamid, Propionamid, Caprolactam und N-Methylpyrrolidon in Betracht, davon besonders bevorzugt Dimethylformamid.

Nach dem erfindungsgemäßen Verfahren lassen sich verschiedene einfach oder mehrfach methoxylierte Benzophenone umsetzen. Dabei ist die Etherspaltung der 2- und 2'-Position besonders leicht zu erreichen, was eine partielle Hydrolyse zu gemischt hydroxylierten/methoxylierten Benzophenonen ermöglicht. Für diese partielle Hydrolyse werden die Reaktionsbedingungen, insbesondere Reaktionsdauer und Temperatur, vorteilhaft von Fall zu Fall in bekannter Weise ermittelt.

Das erfindungsgemäße Verfahren bietet einen vorteilhaften Zugang zu 2,2',4,4'-Tetrahydroxybenzophenon, ein Produkt, das vielfältig verwendbar, z.B. als UV-Absorber ist. Dabei geht man vorzugsweise von 2,2',4,4'-Tetramethoxybenzophenon aus. Dieses Vorprodukt kann auch bis zu 50 % 2-Hydroxy-2',4,4'-trimethoxybenzophenon enthalten. Das Tetrahydroxybenzophenon kann auch aus 2,2'-Dihydroxy-4,4'-dimethoxybenzophenon erhalten werden.

Bei dem erfindungsgemäßen Verfahren ist das Molverhältnis von AlCl₃ zum ganz oder teilweise methylierten Hydroxybenzophenon variierbar. Es wurde jedoch gefunden, daS ein großer Überschuß von AlCl₃ oft zu besonders guten Ausbeuten führt. Dieser Überschuß kann vorzugsweise sogar so groß gewählt werden, daß die Umsetzung praktisch in einer AlCl₃-Schmelze durchgeführt wird. Das oben genannte Molverhältnis beträgt vorzugsweise 5 bis 30, besonders bevorzugt 10 bis 20. Bei Benzophenonen mit wenigen Methoxysubstituenten, z.B. einem oder zwei, kann auch ein geringeres Molverhältnis, z.B. von 1,5 bis 3 ausreichend sein.

Erfindungsgemäß wird der Mischung eine Verbindung aus der Klasse der Harnstoffe oder Carbonsäureamide zugegeben. Das Molverhältnis von AlCl₃ zu diesen Verbindungen wird so gewählt, daß die Mischung bei der Reaktionstemperatur gut rührbar und weitgehend homogen bleibt. Im allgemeinen wird dies durch ein Molverhältnis von 0,5 bis 20, bevorzugt durch ein Molverhältnis von 1 bis 10 erreicht.

Die bevorzugte Reaktionstemperatur hängt vom Molverhältnis AlCl₃/Harnstoff- oder Carbonsäureamidverbindung sowie vom gewünschten Produkt ab. Zur teilweisen Methylierung wird vorteilhaft bei niederer, zur vollständigen Methylierung meist bei etwas höherer Temperatur umgesetzt. Die Temperatur liegt im allgemeinen zwischen Raumtemperatur (20°C) und 160°C, vorzugsweise bei 30 bis 80°C, besonders bevorzugt bei 40 bis 60°C.

Eine bevorzugte Reaktionsführung beginnt mit einer Vermischung von AlCl₃ und der Harnstoff- oder Carbonsäureamidverbindung bei 100 bis 160°C, vorzugsweise bei 140 bis 160°C. Die Mischung wird dann auf 20-50°C abgekühlt, das Edukt eingerührt und dann bei 40-60°C die Umsetzung durchgeführt.

Der Druck ist für die Reaktion kein kritischer Parameter. Im allgemeinen wird die Umsetzung bei Normaldruck vorgenommen, eine Druckerhöhung kann z.B. bei der Verwendung leicht flüchtiger Carbonsäureamide vorteilhaft sein.

Nach der Umsetzung mit AlCl₃ wird die Reaktionsmischung zur Hydrolyse in bekannter Weise mit Wasser versetzt. Vorteilhaft ist der Zusatz von Aktivkohle zur Hydrolysemischung um z.B. thermische Kondensationsprodukte zu binden.

Die Aufarbeitung der Hydroxybenzophenone aus dem Gemisch erfolgt in bekannter Weise z.B. durch Filtration, Ansäuern, Absaugen des ausgefallenen Produkts, Waschen und Trocknen.

Nach dem erfindungsgemäßen Verfahren werden Hydroxybenzophenone von großer Reinheit (oft über 99 %) und in guter Ausbeute erhalten (oft über 90 % d.Th.).

Die erfindungsgemäß hergestellten Hydroxybenzophenone finden bevorzugt Verwendung als UV-Absorber z.B. für Kunststoffe und Lacke oder als Vorprodukte für solche UV-Absorber. Aufgrund ihrer hohen Reinheit eignen sie sich besonders als UV-Absorber in Kosmetika wie Salben, Ölen, Lotionen, Sonnenschutzmitteln und Tabletten, Parfüms, Rasierwasser usw.

### Beispiele

### Beispiel 1 (Vergleichsbeispiel): Herstellung von 2,2',4,4'-Tetrahydroxybenzophenon gemäß JP-A-58 216 139

Eine Mischung aus 300 ml o-Dichlorbenzol, 117,4 g (0,88 mol) AlCl₃ und 60,5 g (0,20 mol) 2,2',4,4'-Tetramethoxybenzophenon wurde auf 115°C aufgeheizt. Bereits ab 70°C setzte sich eine feste Masse über dem Lösungsmittel ab, die Mischung war nicht mehr rührbar. Nach 3,5 Stunden wurde das o-Dichlorbenzol abdekantiert, der feste Rückstand mechanisch zerkleinert und zusammen mit dem Lösungsmittel auf 1 l Wasser gegeben. Anschließend wurde 3 Stunden bei 25°C gerührt, der Niederschlag abgesaugt, mit Wasser gewaschen und bei 125°C im Vakuum getrocknet.
- Ausbeute:: 44,5 g Rohprodukt
- Schmelzpunkt:: 193-198°C.

Nach Umkristallisation aus Wasser wurden 37,2 g Produkt erhalten.
- Schmelzpunkt:: 195-199°C
- Reinheit (HPLC):: 97,2 %.

### Beispiel 2: Herstellung von 2,2',4,4'-Tetrahydroxybenzophenon

In 120 g (1,64 mol) Dimethylformamid wurden 440 g (3,30 mol) AlCl₃ so eingerührt, daß die Temperatur unter 160°C blieb. Nach Abkühlen auf 30°C wurden in die gut rührbare Mischung 60,5 g (0,20 mol) 2,2',4,4'-Tetramethoxybenzophenon eingetragen. Die Mischung wurde 4,5 Stunden bei etwa 50 bis 52°C gerührt und dann in 1,8 l Wasser, dem 3 g Aktivkohle zugesetzt wurden, gegeben. Das Hydrolysat wurde bei ca. 95°C filtriert, dem Filtrat wurden dann 65 g konz. Salzsäure zugegeben. Der ausgefallene Niederschlag wurde bei 25°C abgesaugt, mit Wasser gewaschen und bei 125°C im Vakuum getrocknet.
- Ausbeute:: 45,5 g
- Schmelzpunkt:: 198-200°C
- Reinheit (HPLC):: 99,2 %.

### Beispiel 3: Herstellung von 2,2',4,4'-Tetrahydroxybenzophenon

82,2 g (0,30 mol) 2,2' -Dihydroxy-4,4'-dimethoxybenzophenon wurden mit 600 g AlCl₃ (4,50 mol) und 180 g (2,47 mol) DMF (Dimethylformamid) analog zu Beispiel 2 umgesetzt und das Produkt wurde wie beschrieben aufgearbeitet.
- Ausbeute:: 68,0 g
- Schmelzpunkt:: 198-200°C
- Reinheit (HPLC):: 99,5 %

### Beispiel 4: Herstellung von 2,2',4,4'-Tetrahydroxybenzophenon in Gegenwart von Harnstoff

54,8 g 2,2'-Dihydroxy-4,4'-dimethoxybenzophenon (0,20 mol) wurden mit 400 g AlCl₃ (3,0 mol) und 120 g (2,0 mol) Harnstoff analog zu Beispiel 3 umgesetzt und das Produkt wurde wie beschrieben aufgearbeitet.
- Ausbeute:: 40,3 g
- Schmelzpunkt:: 198-200°C
- Reinheit (HPLC):: 99,3 %

## Patentansprüche

1. Verfahren zur Herstellung von hydroxylierten Benzophenonen aus ganz oder teilweise methylierten Hydroxybenzophenonen durch Umsetzung dieser Methylether mit AlCl₃ und anschließende Hydrolyse mit Wasser, dadurch gekennzeichnet, daß man die Umsetzung mit AlCl₃ in Gegenwart einer oder mehrerer Verbindungen aus der Klasse der Harnstoffe oder Carbonsäureamide durchführt.

2. Verfahren nach Anspruch 1, dadurch gekennzeichnet, daß man als Carbonsäureamid Dimethylformamid einsetzt.

3. Verfahren nach Anspruch 1, dadurch gekennzeichnet, daß man die Umsetzung in Gegenwart von Harnstoff durchführt.

4. Verfahren nach den Ansprüchen 1 bis 3 zur Herstellung von 2,2',4,4'-Tetrahydroxybenzophenon.

5. Verfahren nach den Ansprüchen 1 bis 4, dadurch gekennzeichnet, daß das Molverhältnis von AlCl₃ zum ganz oder teilweise methylierten Hydroxybenzophenon 5 bis 30 beträgt.

6. Verfahren nach Anspruch 5, dadurch gekennzeichnet, daß das Molverhältnis von AlCl₃ zum ganz oder teilweise methylierten Hydroxybenzophenon 10 bis 20 beträgt.

7. Verfahren nach den Ansprüchen 1 bis 6, dadurch gekennzeichnet, daß das Molverhaltnis von AlCl₃ zur Verbindung aus der Klasse der Harnstoffe oder Carbonsäureamide 0,5 bis 20 beträgt.

8. Verfahren nach den Ansprüchen 1 bis 7, dadurch gekennzeichnet, daß das Molverhältnis von AlCl₃ zur Verbindung aus der Klasse der Harnstoffe oder Carbonsäureamide 1 bis 10 beträgt.

9. Verfahren nach den Ansprüchen 1 bis 8, dadurch gekennzeichnet, daß man die Umsetzung bei Temperaturen von 30 bis 80°C durchführt.
